# EUROPEAN PATENT APPLICATION

(11) **EP 1 958 602 A1**
(43) Date of publication of application: **20.08.2008**
(21) Application number: 07102196.8
(22) Date of filing: 13.02.2007
(51) Int. Cl.: A61F 13/15

(54) **Elasticated Absorbent Article**

(71) Applicant: THE PROCTER & GAMBLE COMPANY, Cincinnati, Ohio 45202 (US)
(72) Inventor: Ponomarenko, Ekaterina Anatolyevna, 65812, Bad Soden (DE); Wciorka, Maja, 65824, Schwalbach (DE); Martynus, Cornelia Beate, 61130, Nidderau (DE); Brown, Tina, Cincinnati, OH 45241 (US)
(74) Representative: McGregor, Judit Ester

(57) **Abstract**

The present invention relates to diapers having a specific elasticated anal/ genital cuff, also referred to as elasticated topsheet, and having a fully stretched length of less than 465 mm, suitable for babies of typically up to 9 kg.

## Description

### FIELD OF THE INVENTION

This invention is directed to absorbent articles, such as infant diapers, having a specific elasticated anal/ genital cuff or a topsheet with at least one opening for receiving feces.

### BACKGROUND OF THE INVENTION

Many absorbent articles have been proposed with specific design features to accept or store feces and to reduce feces escaping the article or soiling of the wearer's skin. In particular fluid feces has this problem, since it is very mobile on the topsheet and easily moves from one side to another and easy escapes the diaper's leg portions or leg cuffs.

One type of articles proposed has a topsheet or anal/ genital cuff that has a large opening to receive feces, such as disclosed in U.S. patent No. 4,892,536 issued to DesMarais and 4,990,147 issued to Freeland. Subsequent developments have lead to a diaper that has elastic bands along the opening that diverge towards the front and back, beyond the opening, to better align the opening with the anus and genitals in use, and to better keep the article in place during use, such as described in EP1201212-B.

However, the inventors have found that diapers for smaller babies, which have a very sensitive skin, move less and have typically more liquid feces, may require a different approach to the one suggested in EP1201212-B.
The inventors found that it is desirable for some smaller babies that the topsheet or cuff is more comfortable and/ or creates very limited or no skin marking, and that thus it may be desirable that the article has reduced elastic forces in use. However, the article should still be such that the opening(s) is aligned with the anus at least.

The inventors have now found a way to solve this problem, by providing a topsheet or anal/ genital cuff containing elasticated areas that provide a specific force profile. The articles of the invention are such that the correct alignment of the opening(s) can be obtained whilst being comfortable to wear for the baby.

### SUMMARY OF THE INVENTION

The invention relates to an absorbent article, e.g. diaper, having a backsheet, an absorbent core and an elasticated anal and/ or genital cuff or topsheet, comprising elasticated regions, said cuff or topsheet defining at least one opening to receive fecal material, each having a longitudinal direction, length and longitudinal axis (Y), and a transverse direction, width and transverse axis (X),
whereby in 100% stretched state said article has a length (in longitudinal direction) of 47 cm or less, or typically 46 cm or less, and whereby said article and/ or said elasticated cuff or topsheet has a second unload force at 150% elongation state of at least 0.10N and a second unload force at 200% elongation state (strain) of at least 0.20N or at least 0.3N. In a preferred embodiment, the second unload force at 200% elongation state (strain) is less than 0.9N or less than 0.8 N or 0.7 or less.

In one embodiment, the backsheet is non-stretching and has a maximum length equalling the length of the article in stretched state.

Preferred articles herein have a very soft cuff or topsheet, namely made of specific nonwoven material described herein. The elastic bands or strands may be attached to the nonwoven material by a preferred pattern described herein, to further reduce the risk of skin marking.

The absorbent article is preferably a baby or infant diaper.

### BRIEF DESCRIPTION OF THE FIGURES

Figure 1 is a plan view of a preferred disposable diaper configuration of the present invention.
Figure 2 is perspective view of a preferred disposable diaper of the present invention.

### DETAILED DESCRIPTION OF THE INVENTION

As used herein, the following terms have the following meanings.
As used herein, 'absorbent article' means any article that can absorb body fluids and is suitable to be placed in close to or against the genitals of the user, including in particular and adult or infant diaper and so-called training or pull-up pants.
As used herein 'front region' and 'back region' refer to the two regions, which are in use, respectively, closest to the front of the wearer and the back of the wearer, each having half the longitudinal length of the article or topsheet.

As used herein, the term 'void space' is a cavity in the article present in at least the relaxed state, which serves to accept and contain bodily exudates such as fecal material, typically being at least 5 cm³ in relaxed state.
As used herein, 'longitudinal' is the direction running substantially parallel to the maximum linear dimension of the component, typically to the longitudinal axis of the article, and includes directions within 30° of this parallel, unless otherwise stated.
The 'transverse' direction is orthogonal to the longitudinal direction and in the same plan of the majority of the article and the longitudinal axis and includes directions within 30° of the orthogonal, unless otherwise specified.
As used herein, 'elasticated' means typically, that the component consists of or comprises elastic material, which is elastic in at least one direction. 'Non-elasticated' when used herein means that the component does not comprise any elastic material.
As used herein, 'opening (31) in the topsheet' means an area completely circumscribed by the topsheet or sheet components thereof, but where no topsheet material is present, and which is large enough to receive fecal material, typically being at least 2 cm long or wide, or having a surface area of at least 2 cm².
The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".
The dimensions herein are either determined at 75% article length, if so indicated, or at the fully stretched article length, unless otherwise indicated.

The present invention relates typically to baby diapers that have a article length, in fully stretched state of 47 or less or 46.5 cm or less, preferably 46 cm or less, or 45.5 cm or less or 45 cm or less, and typically more than 30 cm, or more than 35 cm or m ore than 37 cm. This is measured in the fully stretched state of the article, by stretching it to its full length and width on a stretch board. In one typical embodiment when the backsheet is non-stretching, this full length is determined by the length of the backsheet and the backsheet has then typically the same length as the diaper length mentioned above. The topsheet or cuff herein may also be of the same maximum length as specified above.

The present invention provides a wearable absorbent article having a topsheet or anal and/ or genital cuff, defining at least one opening in close proximity to the anus and/ or genitals (herein any features described for the topsheet are equally applicable to the cuff, and vise versa, unless otherwise specifies). The opening may be a (single) elongate split opening. The opening is in communication with a void space, which is suitable to receive and store bodily exudates. In a preferred embodiment of the invention, the article herein has a topsheet or cuff defining an opening, preferably an elongated split opening, leading to a void space, whereby the opening has longitudinal side edges and along each side edge, or at least part thereof one or more elasticated regions. The elasticated regions maintain improved longitudinal and transverse alignment, as well as Z-direction proximity with a point of discharge on a wearer, e.g. stays in close proximity, preferably contact with the wearer.

In one embodiment, the topsheet or cuff defines one such opening with a maximum length, in fully stretched state, of from 40% to 80%, or even more preferably about 55% to 70%, of the total fully stretched length of the absorbent article. This is measured in the fully stretched state of the article, by stretching it to its full length and width on a stretch board, as above.

The maximum width and/ or a crotch width (at the exact x-axis, transverse ax herein may for example be from 5% to 30%, or more preferably 10% Sample aligned at back edge of sample holder; Sample fully stretched out in MD and CD over sample holder area. 15% average width of the absorbent articles width along the transverse axis thereof average width of the absorbent articles width along the transverse axis thereof herein by determining the fully stretched, 100% article length and then stretching the article to its 75% (+/- 1 %) stretched article length, by placing each transverse end edge of the article, completely stretched in width and length direction of said edge, in a horizontal sample holder, said pair of sample holders comprising fixing means, e.g. Velcro, to fix to the article, each holder having a length corresponding to the width of the article's transverse edge (in fully stretched state) and a width placed over the transverse edge of 50 mm. Then the maximum width (at 75% longitudinally stretched state) can be determined and / or the width and the transverse x-axis.

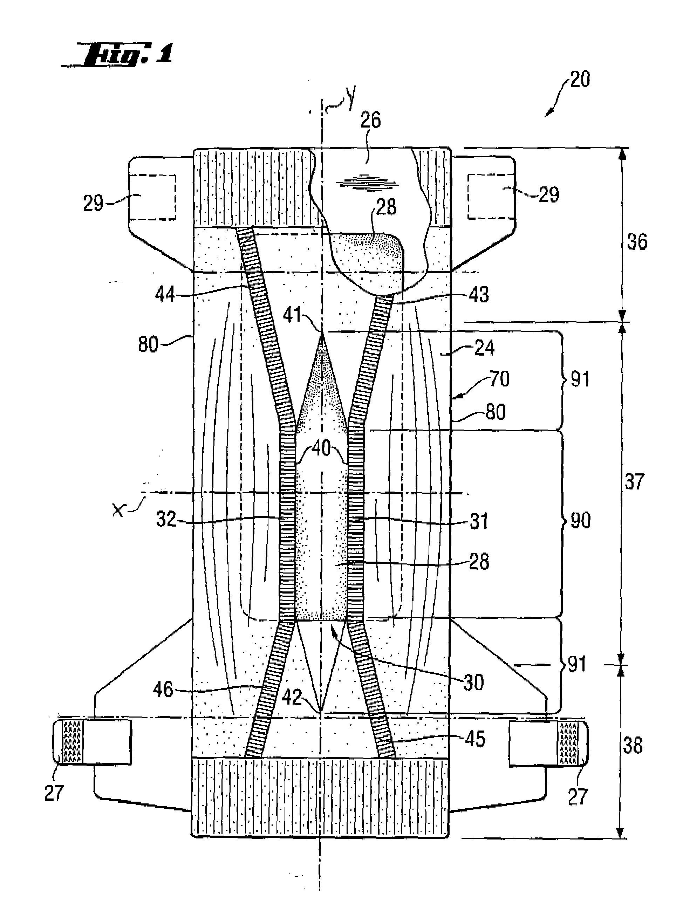

The opening may have transverse end edges parallel to the transverse axis of the article, but preferred may be that one or both edges comprise each two edges, connected by an angle, such that the length of the opening on the longitudinal centre line thereof, e.g. Y-axis, is more than the length of the opening on either side thereof (e.g. defining a triangular end portion to the opening, as shown in Figure 1).

The article, e.g. diaper, and/ or the elasticated cuff/ topsheet comprises elasticated areas and either or both has a certain force profile, namely:
said article, or elasticated cuff or topsheet has a second unload force at 150% elongation state, as defined herein, of at least 0.10N and a second unload force at 200% elongation state of at least 0.2 or at least 0.3N. At 200% elongation state, a second unload force of less than 0.9N, or less than 0.8 N, or 0.7 N or less, or 0.6 or less may be preferred, or in one embodiment herein even essential, The second unload force and 100% elongation state nay for example be between 0.3N and 0.45N.

In another embodiment, said absorbent article, e.g. diaper, or elasticated cuff or topsheet has a second unload force at 150% elongation state, as defined herein, of at least 0.15N and a second unload force at 200% elongation state of at least 0.40N, and typically less than 0.9 N less than 0.8N, or 0.7N or less, or 0.6N or less, at 200% elongation state;

In one embodiment, the article, cuff or topsheet thereof may for example have a second unload force at 300% elongation state of at least 0.9N or at least 1.0N, but preferably 2.0N or less, or 1.5N or less.

The article, cuff or topsheet may have a first load force at 300% elongation state, as defined herein, of less than 3.0N, preferably less than 2.8 N or less than 2.7N, and optionally the article, e.g. diaper, cuff or topsheet may have a first load force at 300% elongation state, as defined herein, of at least 0.9N, or at least 1.0N.

The elasticated regions are preferably positioned along the two longitudinal edges of the opening (so that each edge has at least one elasticated region), extending from said opening towards the first (front) and second (back) waist region, preferably such that the end portions of the elastic regions can be attached or joined to the waist region. Thus, the elasticated regions are preferably longer than the opening(s), both in relaxed as in stretched state. Preferred is that the elastic region is positioned over at least 80% of the length of the topsheet.
The length of the elastic region will typically depend on the size of the topsheet and/ or the article. For example, for a size 3 diapers, having a backsheet length or stretched article length as defined herein of 46.5 cm or less, the length of the elastic region in fully stretched state position may be 10 to 30 cm, or even 15 to 25, whilst in stretched position preferably from 20 to 60 or even 25 to 45 or even 30 to 40cm.

Two elastic regions (of a pair of elastic regions) may be mirror images of one another in the Y-axis of the article. They may be such that the pair comprises substantially parallel elasticated areas, positioned along at least part of each of the longitudinal edges of an opening defined by the cuff/ topsheet. Preferred is that the elasticated regions are shaped such that the middle portions of the regions are substantially parallel to one another, whist the end portions, both in the front and back, (at least in relaxed state) bend away from one another (in the plane of the topsheet/ cuff), so that the distance between the end portions of the elastic regions is larger that the distance between the middle portions of the elastic regions. Then, in fully stretched state on a stretch board, as described above, both the two front end portions and the two back end portions of the elasticated regions make typically each an angle with the longitudinal axis of the opening, preferably each angle being between 15° and 35°, preferably from 20° to 30°, or preferably from 24° and 28°; and preferably such that the angle between the end portions is about twice as much, e.g. between 30° and 70° or even between 40° and 60° (in order to measure this, the longitudinal edges of the opening are in fully stretched state placed against one another prior to measuring these angles). This is herein referred to as a X-shape, and a preferred x-shape is exemplified in Figure 1, as described herein after. Thus, the elasticated regions are preferably in the shape of an X, whereby the end portions of the elasticated regions bend away (diverge) from one another, e.g. such that the distance between the end regions of the elasticated regions, both on the front and back side, is larger than the distance between the middle points of both elasticated regions.

Optional may be that, in relaxed state, the elastic regions or part thereof are under an angle with the adjacent topsheet, such that the elastic regions are (also) bending out of plane of the topsheet, bending upwards and away from the void space (under the topsheet).
The topsheet's / cuffs elasticated regions are typically each in the form of an elastic component, such as one or more elastic bands or strands, that are attached in stretched state to a supporting sheet that forms part of the topsheet/ cuff, e.g. a nonwoven sheet as described herein.
Due to the elastic profile of the article, the article may be stored and packed in folded state, typically folded at least twice around transverse folding lines. For example, a preferred diaper herein may be folded twice, around two different transverse lines, to thus obtain a folded diaper of less than ½ of its original unfolded length, e.g. about 1/3 of the original length.

In one embodiment, an elastic region, or elastic laminate portion, formed by attachment of an elastic component to a carrier sheet, e.g. a nonwoven forming (part of the cuff or topsheet) has in relaxed state and in partially stretched state, including at an elongation ε of 0.5 as described herein below, at least one surface with wrinkles that face the user's body and may be in contact with the skin in use. The elastic laminate portion or region may comprise two or more (preferably non-elastic) sheets of material that are attached to either surface of an elastic material, and the laminate portion than typically contains wrinkles on either surface of the elastic laminate portion.
The properties of said wrinkles described herein applies at least to the wrinkles of the body-facing surface of the elastic laminate portion or region but may apply to both surfaces of said laminate or region.
In one embodiment, and at an elongation ε of 0.5, whereby ε = (Lₓ- L_{c}) /L_{c}), said wrinkles are of an average wrinkle height (as measured by the "Primos" method set out below, using PRIMOS equipment) of less than 600 microns, and typically less than 600 but more than 200 microns, or between 550 microns and 300 microns, or up to 530, or even up to 500 microns.
In one embodiment, and at an elongation ε of 0.5, the elastic laminate portions (10) herein may have an average wrinkle density (wrinkles per cm) of from 5 to 10 wrinkles per cm or even from 6 to 10, or even 7 to 10, or possibly only up to 9 wrinkles per cm, as measured with the Primos method set out herein.
The elastic regions or elastic laminate portions herein may have an average maximum elongation εₘₐₓ, (being (Lₛ- L_{c})/ L_{c} whereby Lₛ is the fully stretched length of the elastic laminate portion), of at least 0.8 or even more preferably at least 1.0 or even more preferably at least 1.2, or it may be at least 1.4. These values can be obtained by the method set out in the method section below. It should be understood that the cuff or topsheet may comprise areas (typically of between 0.5 and 2 cm, or 0.8 and 1.5 cm) where an elastic material is attached to the supporting sheet material without providing in that area any elongation of at least 0.5, and there are then possibly not even any wrinkles present. It should be understood for the purpose of the invention that such areas where elastic material is present but that have an elongation of less than 0.5 are not considered part of the elastic laminate portion/ region herein. Such areas may herein be referred to as "attachment portions".
In order to determine and obtain the elongation ε = 0.5 of the elastic region or elastic laminate portion, the elastic region's or elastic laminate portion's absolute contracted length L_{c}, is first determined as follows.
The cuff / topsheet with the elastic laminate portion/ region is removed from the absorbent article, or if possible the elastic laminate portion/ region is removed from the article, either way such that the wrinkle profile and elastic profile (i.e. of the upward facing surface that in use is facing the body of the user) is not changed. The cuff or elastic laminate portion / region is placed as flat as possible on a surface, without applying any elongating or stretching force to it. Then, the absolute contracted length of the elastic laminate portion / region of the cuff is measured. This is herein referred to as the absolute contracted length of the laminate L_{c}.
Then, the length of the laminate portion / region at ε = 0.5 can be calculated, this being 1.5 Lc
(based on : ε = (Lₓ- L_{c}) / L_{c}).
Subsequently, the laminate portion / region can be stretched by the method described herein below to obtain this elongation of 0.5 at 1.5 L_{c}. Then, the wrinkle heights, average wrinkle height and deviations, wrinkle width, distance between wrinkles, average wrinkle density and deviation thereof can be calculated by use of the Primos method, using PRIMOS equipment, as described below.
Typically, the elastic laminate portion/ region has wrinkles of a relatively or substantially uniform wrinkle height (distribution), at least on the body-facing surface of the elastic laminate portion / region. For example, less than 10% or preferably less than 5% of the wrinkles are 800 microns or more, preferably less than 10% or even less than 5% of the wrinkles are 700 or more, or even 650 microns or more; and it may even be possible that 95% or more, or even all wrinkles (about 100%) have a height of less than 600 microns.
Furthermore, it may be preferred that the wrinkle density is substantially uniform throughout an elastic laminate portion e.g. that in no section of 2 cm (in length direction, along the laminate portion) the wrinkle density is more than 12 and that in no section of 2 cm (in length direction, along the laminate portion) the wrinkle density is less than 3, or less than 4. (Preferred may be that in each 2 cm section of a laminate portion the wrinkle density is between 5 and 10, or any of the preferred values described above.
The width of the elastic laminate portions / regions will vary, typically depending on the exact dimensions of the cuff/ topsheet or of the article.
For example, each elastic laminate portion or region may have an average width of about 0.5 to 2 mm or to 1.5 mm.
The elastic materials used herein to form the elastic region/ laminate potion may be very thin, typically having a thickness or caliper (e.g. gauge) of typically up to about 200 microns, or even up to 150 microns or even up to 110 microns, or up to 100 microns and they may need to be at least 20 microns, more preferably at least 40 microns, or even at least 60 microns, as defined herein. Highly preferred materials have a thickness of about 70 to 100 microns.

Preferred elastic components or materials herein include VFE-CD, available from Tredegar, and L-86, available from Fulflex (Limerick, Ireland), or L-89, available from Fulflex, or optionally Lycra.

Preferred is that the opening, positioned in at least the crotch region of the topsheet, is configured such that from 0%, or even from 10%, or even from 20% to 40% or even to 30% of the length of the opening extends from the transverse axis of the article towards the front transverse edge of the article, and the remaining percentage extends from the x-axis towards the back transverse edge of the article.

The dimensions of the opening may vary, depending on the size of the cuff/ topsheet or the article. Preferred may be herein that the absorbent article is a baby diaper, with the article length defined herein, whereby the length of the opening, e.g. in fully stretched state is from 20 to 35cm. The transverse width or average width, measured as described above, of the opening of such articles is preferably from 2 to 6 cm, more preferably 3 to 5 cm or 3.5 to 5 cm.

The anal / genital cuff or topsheet herein may be liquid pervious or impervious, or both in parts. It may be useful that the topsheet is liquid pervious in one direction, but liquid impervious in the opposite direction, e.g. that body fluids may penetrate through the topsheet to the remaining part of the diaper, but that no or limited amounts of fluid can penetrate in reverse direction, towards the wearer's skin. For example, the topsheet may be treated with a chemical such that it is hydrophilic on one side and hydrophobic at the opposite side, as described hereinafter.

Preferably, the topsheet or cuff is made of a hydrophobic material or is treated to be hydrophobic in order to isolate the wearer's skin from liquids contained in remaining part of the diaper. For example, if the topsheet is made of a hydrophobic material, preferably at least the upper surface of the topsheet is treated to be hydrophilic so that liquids will transfer through the topsheet more rapidly. This diminishes the likelihood that body exudates will flow off the topsheet rather than being drawn through the opening of the topsheet. The topsheet or cuff can be rendered hydrophilic by treating it with a surfactant or by incorporating a surfactant into the topsheet. Suitable methods for treating the topsheet with a surfactant include spraying the topsheet material with the surfactant and immersing the material into the surfactant. A more detailed discussion of such a treatment and hydrophilicity is contained in U.S. 4,988,344; U.S. 4,988,345; U.S. Statutory Invention Registration No. H1670, published on July 1, 1997 in the names of Aziz et al.

Any portion of the topsheet or cuff may be coated with a lotion as is known in the art. Examples of suitable lotions include those described in U.S. 5,607,760; U.S. 5,609,587; U.S. 5,635,191; U.S. 5,643,588; WO 95/24173.

The topsheet or cuff may be a nonwoven comprises at least spunbond fibers and for example meltblown fibers and/ or nanaofibers, including for example polyalkylene fibers or derivatives thereof, e.g. polyethylene and/ or polypropylene fibers or bicomponent fibers.

In one embodiment, the topsheet or cuff herein comprises a nonwoven sheet that is a barrier sheet, said topsheet or cuff and/ or said nonwoven sheet having typically a hydrostatic head value (measured with a 49 mN/m liquid with the hydrostatic head test set out herein) of at least 18 mbar, preferably at least 20 mbar, or at least 25 mbar, or at least 28 mbar, or at least 30 mbar, or optionally at least 35 mbar, and optionally less than 50 or less than 45 mbar. A nonwoven sheet, topsheet or cuff herein is considered to have the above hydrostatic head values if it has this value at any part of the material, excluding areas comprising elastic material or edges attached to another material: i.e. the measurement is done on a sample that does not comprise elastic material or edges attached to another material. In one embodiment, the nonwoven sheet or cuff or topsheet has a surface are free of elastics or edges of at least 2.5 cm x 2.5 cm.

The nonwoven sheet of the topsheet or cuffs herein may comprise at least two nonwoven layers or nonwoven single webs, preferably at least two nonwoven layers that each comprise two or more nonwoven single webs. In one embodiment, at least one nonwoven layer or web is a skin-facing nonwoven layer or web (i.e. that in use faces the skin of the user and may contact the skin of the user), said skin-facing nonwoven layer or web having for example a bending rigidity of 20 grams or less, preferably 16 grams or less, or even 14 grams or less or 12 grams or less, as measured with the handle-o-meter rigidity/ softness test set out herein.
Alternatively, or in addition the nonwoven sheet as a whole (so not just the skin-facing layer or web thereof) of the topsheet or cuff as may have a bending rigidity of less than 35 grams, or less than 30 grams or less than 25 grams or less than 20 grams or less than 18 grams. (A nonwoven sheet, nonwoven layer, topsheet or the cuff herein is considered to have the above bending rigidity values if it has this value at any part of the material, excluding areas comprising elastic material or edges attached to other materials (these latter should not be included in the test)). The bending rigidity as referred to herein, and measured with the method herein, is the rigidity of said nonwoven layer, nonwoven web or nonwoven sheet in any direction, unless specified otherwise.

In one embodiment, the nonwoven sheet comprises on its skin-facing surface a nonwoven web or a nonwoven layer that has on its skin-facing surface a nonwoven web, said nonwoven web comprising fibers with an average fiber direction, and said nonwoven layer or nonwoven sheet has a bending rigidity of the values specified above, in said fiber direction. Preferred skin-facing webs with fibers with an average fiber direction are spunbond webs. Thus, the nonwoven sheet herein has preferably on its skin-facing surface a nonwoven web, which may be part of a nonwoven layer, and that comprises fibers with an average direction, such as a spunbond web with the above bending rigidity in the fiber direction. The average fiber direction may typically be the machine direction (MD) of the absorbent article.

In one embodiment, the nonwoven sheet of the topsheet or cuff comprises spunbond and meltblown webs, and/ or spunbond and nano-fiber webs.
It may be preferred that the topsheet or cuff comprises a nonwoven web that comprises nano-fibers that have an average diameter of 1.0 microns or less. Preferred may be that the nonwoven sheet comprises two or more nonwoven layers, whereof one or more or each comprise a nonwoven web that comprise such nano-fibers. The nonwoven sheet or layer thereof may for example comprise at least 2 g/m² of nano-fibers, or at least 3 g/m² or at least 5 g/m² of nano-fibers. The nano-fibers may have an average diameter of 0.8 microns or less, or 0.6 microns or less. The nano-fibers may be made by known melt fibrillation methods or melt film fibrillation methods, such as described in US6,315,806 and US6,695,992. Preferred nano-fiber webs and layers are described in co-pending application WO2005/103355.

Preferred may be that the topsheet or cuff comprises at least one nonwoven layer that is a laminate of nonwoven webs, or even that it comprises two such nonwoven layers that may be laminated together or that may be not fully laminated, e.g. having an attachment are of less than 60% or less than 40% or being for example only attached to one another by the edges (and optionally by the elastic component(s) that may be placed between said layers).
In one embodiment, at least one nonwoven layer, or each nonwoven layer comprises a nonwoven web of meltblown fibers, typically present at a weight level of at least 5 g/ m² by weight of the nonwoven layer, or for example at least 5.7 g/ m², or at least 7 g/ m², but typically less than 20 or 15 g/m²by weight of the nonwoven layer.
It may be preferred that the basis weight of the nonwoven sheet is 45 g/ m² or less, or 40 g/m2 or less or 35 g/m² or less or 30 g/m² or less. Preferred may be that the nonwoven sheet comprises only two nonwoven layers, each comprising two or more nonwoven webs. Preferred may be that the basis weight of each of the nonwoven layers present in said nonwoven sheet is 24 g/m² or less, or 22 g/m² or less or 18 g/m² or less.

The nonwoven sheet may comprise a hydrophobic agent, such as a wax. The nonwoven sheet or one or more layer thereof may also comprise a barrier agent, also referred to as masking facilitating agent, as described below.
The nonwoven sheet herein may have on one surface, e.g. the skin-facing surface a spunbond nonwoven web. Preferred is that the nonwoven sheet comprises nonwoven layers comprising spunbond webs (S) and meltblown webs (M) and/ or nano-fiber webs (N), whereby it may be preferred that the outer surface of the nonwoven sheet is formed by a spunbond web.

Preferred are herein are: a nonwoven sheet comprising a 17 or 22 gsm (g/m²) SMMMS nonwoven layer attached to (but not laminated to) another 17 or 22 gsm SMMMS nonwoven layer (whereof for example the meltblown level of each layer is 5.7 or 7.3 gsm respectively), including a nonwoven sheet comprising 22 gsm SMMMS nonwoven layer, with for example 7.3gsm meltblown fibers, attached to 17 gsm SMMMS or SMMS nonwoven layer, comprising for example 5.7 gsm meltblown fibers; a nonwoven sheet comprising a 17 gsm or 22 gsm SMS or SNS nonwoven layer, attached to another 17 gsm or 22 gsm SNS or SMS nonwoven layer; a 17 or 22 gsm SMMS nonwoven layer attached to a 17 or 22 gsm SMMS or SMMMS nonwoven layer (comprising for example 3 gsm or 5.7 gsm (for SMMS) or 7.3 gsm meltblown fibers per layer).

In one embodiment of the invention, the absorbent article may also comprises a genital coversheet that is present under, in or above the opening in the topsheet, typically only that part of the opening that is in close proximity with the genitals during use, i.e. the front region of the opening. Preferably, the maximum length of the part of the genital coversheet that is present above, in or under the opening genital coversheet is (in fully stretched flat state) 10% to 50% of the maximum length of the opening, preferred 10% to 30%, or more preferably 13% to 28% or even more preferably 17% to 27%. In other words, at the most 50% of the maximum length of the opening is 'covered' by the longest part of the genital coversheet, but at least 10% of the maximum length of the opening is covered by the longest part of the genital coversheet.
The genital cover sheet may have a substantially transverse edge above, in or under the opening, which is not straight, but preferably curved or V-shaped (arrow shaped), having the centre point of the curved edge or of the V-shaped edge closer to the front of the article than the remaining part of the curved edge or V-shaped edge. Then, the length of the part of the genital coversheet from this centre point to the front of the opening is preferably 10% to 30% of the maximum length of the opening (5), more preferably 15% to 25%, or even to 20%.

The topsheet or cuff is positioned adjacent the body facing surface of the absorbent core, and the longitudinal edges are preferably joined or attached to the longitudinal edges of the backsheet, by any attachment means known in the art. In one embodiment of the present invention, the topsheet and the backsheet are attached directly to each other in some locations and indirectly joined together by the leg cuffs in other locations, i.e. by directly joining each to the leg cuffs of the diaper.

In a preferred embodiment herein the topsheet or cuff has longitudinal and/ or transverse folds, and in that case. In one embodiment, the cuff or topsheet width, including the width of the opening, in the crotch (along transverse axis, intersecting centre point of the longitudinal axis) is more than the width of the core in that point.

The core width in said crotch point may be for example from 7 to 10 cm, or from 7.5 or 8 to 9.5 cm.

The width of the topsheet or cuff including the width of the opening may in the crotch point be even more than the width of the backsheet in said crotch point.

The diaper herein also comprises a, typically liquid impervious, backsheet. Preferably also an absorbent core is present, which is preferably positioned between at least a portion of the topsheet or cuff and the backsheet. In preferred embodiments, the backsheet is impervious to liquids (e.g., urine) and comprises a thin plastic film such as a thermoplastic film having a thickness of about 0.012 mm (0.5mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials, which permit vapors to escape from the diaper while still preventing exudates from passing through the backsheet.

There may be a secondary topsheet or core-cover sheet present, positioned between the core and the cuff/ topsheet. It may be beneficial if the diaper comprises an apertured sheet or apertured formed film underneath the topsheet or cuff herein, and positioned on the absorbent core, as also described below.

The diaper may have a pair of elasticated barrier and/ or leg cuffs. The diaper has a first or front waist region a second or back waist region, opposed to the first waist region, and a crotch region, located between the first waist region and the second waist region. The crotch region is typically that portion of the diaper which, when worn, is between the legs of the wearer. The waist regions of the diaper, when worn, typically gather or encircle the waist of the wearer and are generally at the highest at the highest elevation of the article, when the wearer is in the standing, upright position.
It may be beneficial that the backsheet and topsheet in the waist region are free from any elastic material that has transverse stretch around the waist, e.g. free of an elastic waist band, to make the diaper more comfortable.
The diaper preferably has a fastening system, typically joined to the waist region. Preferred fastening systems comprise a fastening ears and tabs attached in or to the back waist region and landing zones in the front waist region. The fastening system preferably maintains the first waist region and the second waist region in a touching or overlapping configuration so as to provide lateral tensions or force line about the circumference of the diaper to hold the diaper on the wearer. The fastening system preferably comprises tape tabs and/or hook and loop fastening tabs, although any other known fastening means are generally acceptable. Some exemplary fastening systems are disclosed in U.S. 3,848,594; U.S. 4,662,875; U.S. 4,846,815; U.S. 4,894,060; U.S. 4,946,527; U.S. 5,151,092; and U.S. 5,221,274; and U.S. Pat. No. 4,963,140.

The absorbent core may comprise any absorbent material which is generally compressible, conformable, non-irritating to the wearer's skin, and capable of absorbing and retaining liquids such as urine and other certain body exudates, such as comminuted wood pulp, creped cellulose wadding; melt blown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials. Exemplary absorbent structures for use as the absorbent core are described in U.S. 4,610,678; U.S. 4,673,402; U.S. 4,834,735; U.S. 4,888,231; U.S.5,137,537; U.S. 5,147; U.S. 5,260,345; U.S. 5,387,207; and U.S. 5,625,222.
Preferred may be that the absorbent core comprises at least one layer that comprises at least 80% by weight of the content of that layer of superabsorbent polymers, and less than 20% by weight of absorbing pulp fibers. It may be preferred that the core comprises adhesive material, e.g. non-absorbing adhesive fibers, and superabsorbent polymers.

The diaper may also include a 3-D sub layer disposed between the topsheet and core, capable of accepting, storing or immobilizing bodily exudates. Suitable materials for use as the sub layer may include 3-D apertured films, mentioned above, or large cell open foams, macro-porous compression resistant non woven highlofts, large size particulate forms of open and closed cell foams (macro and/or microporous), highloft non-wovens, polyolefin, polystyrene, polyurethane foams or particles, structures comprising a multiplicity of vertically oriented looped strands of fibers, absorbent core structures described above having punched holes or depressions, and the like. (As used herein, the term "microporous" or refers to materials that are capable of transporting fluids by capillary action. The term "macroporous" refers to materials having pores too large to effect capillary transport of fluid, generally having pores greater than about 0.5 mm in diameter and more specifically, having pores greater than about 1.0 mm in diameter.)
The diaper preferably further includes leg cuffs that provide improved containment of liquids and other body exudates. Leg cuffs may also be referred to as leg bands, side flaps, barrier cuffs, or elastic cuffs, as described in; U.S. 3,860,003; U.S 4,808,178 and 4,909; U.S. 4,695,278 and 4,795,454.
The diaper may include a topical lotion for the skin of the wearer, and/ or a topical adhesive or body adhering composition which acts to hold the opening further in place during use. Typically, this is comprised by the topsheet or part thereof, so as to further improve the alignment of the opening in the topsheet with for example the anus of the wearer. The topical adhesive may be located on the topsheet, or the body adhering composition may (also) be integral with the material making up the topsheet or other element of the absorbent article or may be a separate material disposed directly or indirectly on all or any portion of the absorbent article. Further, the body adhering composition may be disposed on any portion of the absorbent article in any pattern or configuration including, but not limited to lines, stripes, dots, and the like. In one preferred embodiment, the topical adhesive is present on the elasticated regions along the edges of the opening. Suitable body adhesives are known in the art.
Preferably, the diaper includes a thermally activatable adhesive, which acts to hold the article or some portion thereof in place during use.

### Preferred processes to make the article of the invention

Any method may be used to attach the elasticated region, e.g. bands, strands to the topsheet or cuff material (e.g. nonwoven) herein. They may be attached prior to formation of the opening, or after formation of the opening. They may be attached by heat bonding and gluing methods, as known in the art. Preferred methods are described above.

In a non-limiting example, two elastic bands of 2 L-89 elastic material, available from Fulflex, with (in relaxed state) a thickness of about 70 microns, a width of 20 mm and a length of 13 cm, are obtained MA. In relaxed state, a slit opening is cut in a piece of nonwoven.

One elastic band is glued in a stretched state to each longitudinal edge of the opening of the non-woven. This is done such that the middle 10 cm of the elastic band is stretched to about the length of the non-woven, minus the length needed to attach the elastic film on both sides towards the transverse end edges (for example 2x2.5cm).

Each elastic band is glued on the non-woven in a manner that the ends of the bands bend away from the ends of the opposing elastic band, in the shape of an X. This is done such that, in after application, the distance between to the ends of the (end portions or regions of the) strands is 70 mm in contracted state, whilst the distance between the center point of the strands is only 30 mm, in contracted state. The angles between the end portions of the strands are as mentioned above.

The topsheet may be used on a Pampers Premium Size 3 diaper, designed for a baby weight range of 4 to 9 kg, or may replace the topsheet of such a diaper. Thereto, the topsheet with the elastic bands are attached to the front and back waistbands.

### Preferred articles of the figures

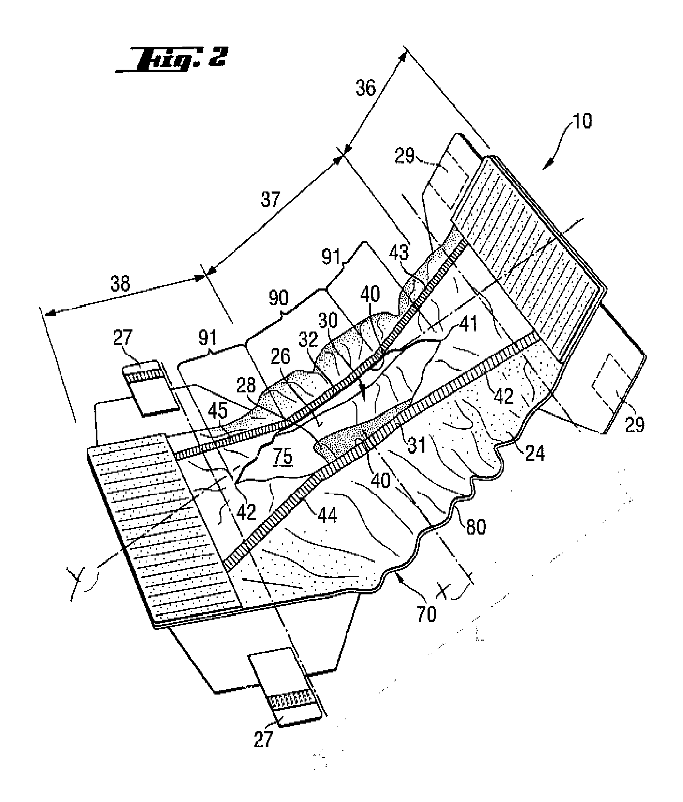

Preferred articles of the invention are now being described with reference the figures 1 and 2
Figure 1 is a plan view of the diaper 20 in its stretched state with portions of the structure being cut away to more clearly show the underlying structure of the diaper 20 and with the portion of the diaper 20 which contacts the wearer facing the viewer. One end portion of the diaper 20 is configured as a first waist region 36 of the diaper 20. The opposite end portion is configured as a second waist region 38 of the diaper 20. An intermediate portion of the diaper 20 is configured as a crotch region 37 that extends longitudinally between the first and second waist regions 36 and 38. The waist regions 36 and 38 generally comprise those waist portions of the diaper 20 which, when worn, encircle the waist of the wearer. The waist regions 36 and 38 includes elements which can gather about the waist of the wearer to provide improved fit and containment, or which typically can gather around the waist and can be fastened around the waist by use of fastening means, such as tabs 27, which are fastened to landing zones 29.
However, in one preferred embodiment, the waist region may not comprise the elastic transverse band, or waist band feature, as for example shown in Figure 1.
The crotch region 37 is that portion of the diaper 20 which, when the diaper 20 is worn, is generally positioned between the legs of the wearer.

The diaper 20 comprises topsheet 24, a liquid impervious backsheet 26, and an absorbent core 28 encased between the topsheet 24 and the backsheet 26. The topsheet may include regions of reduced permeability to fecal material.
The topsheet 24 comprises a slit opening 30, along the longitudinal axis x of the diaper 20, which is configured to receive fecal exudates and isolate at least a portion of the exudates from the skin of the wearer.
The topsheet 24 may be fully or partially elasticated. In figure 1, the topsheet24 is partially elasticated by the provision of elastic bands 31 and 32, which have an X-shape.
The slit opening 30 is located in the topsheet 24 such that the fecal exudates pass through the opening into a void space formed between the topsheet 24 and the absorbent core 28 and/or other underlying layers such as sub layers, acquisition layers and the like. The void space entraps or encapsulates bodily waste. It is also contemplated that the void space may be formed between two elements of the diaper 20, including but not limited to the topsheet 24 and the backsheet 26, the acquisition layer and the core 28, the core 28 and the backsheet 26, etc
The slit opening 30 in the topsheet 24 is located in alignment with at least the wearer's anus during use. Preferably, the slit opening 30 in the topsheet 24 is located in a target zone of the diaper. The target zone is that portion of the diaper, which is configured to directly receive the insult of fecal matter from the wearer and is generally located in the crotch portion of the diaper.
The slit opening 30 in the topsheet 24 is generally disposed in the target zone along the longitudinal axis x and is defined by two opposing longitudinally extending side edges 40, a front edge 41 and a back edge 42. The front edge 41 is generally located in the crotch region 37 of the diaper 20 towards the first, front region 36, or in the first waist region 36 itself, while the back edge 42 is located in the crotch region 37 near the second waist region 38, or in the second waist region 38 itself. The slit opening 30 includes a length in the longitudinal direction parallel to the longitudinal axis x of the diaper and a width in the lateral direction which is parallel to the lateral axis y of the diaper 20. The length of the slit opening 30 is within the ranges specified above.
The diaper 20 preferably also includes a fastening system, typically including at least one engaging component (or fastener of male fastening component) 27 and at least one landing zone 29 (female fastening component), such as hook-loop type fastening systems. The diaper 20 may also include such other features as are known in the art, including leg cuffs, front and rear ear panels, waist cap features, elastics and the like to provide better fit, containment and aesthetic characteristics. Such additional features are well known in the art and are described in U.S. Pat. No. 3,860,003; and U.S. Pat. No. 5,151,092 which are incorporated by reference herein.
The diaper 20 of the present invention includes an elastically foreshortened topsheet including elasticated regions 31 and 32, along at least portions of the longitudinal edges 40 of the slit opening 30. The elastic regions 31 and 32 ensure that the opening 30 of the diaper 20 is positioned and remains positioned in the gluteal groove of the buttocks, including the perianal region.
The edge 40 of the slit opening may be held against the wearer ('s skin) allowing the feces to penetrate the slit opening 30 without deflection, via only the elastic forces supplied by the elastic regions 31 and 32, or optionally additionally by use of a body adhering composition, as described above.
The elasticated regions 31, 32 may be formed by bonding pre-stretched elastic bands along the longitudinal edges 40 of the slit opening 30, by the method described herein.
The elastic regions 31, 32 extend from the slit opening 30 in the direction of the waist regions, preferably in a X-shape, with front elastic regions 43 and 44 and/ or back elasticated regions 45 and 46. In stretched state, preferred maximum distance between the elastic regions 32 and 31 is at least 150% of the minimum distance between the elastic regions 31, 32.
Figure 2 shows how the diaper 20 in contracted state. The elastic regions 31, 32 are in the shape of an X, and extend along the slit opening 30 into the waist regions 36, 38, and are attached to the waistbands. The elastic regions 31, 32 have an angle with the joining topsheet 24, such that the elastic regions bend away from the void space and the backsheet 26 and core 28.
The elastic regions 31, 32 preferably extend from the slit opening 30, as can be seen in figure 1, in the direction of the waist regions, preferably in a X-shape, with front elastic regions (or portions) 43 and 44 and/ or back elastic regions (or portions) 45 and 46.

Unlike the slit opening 30 in figure 1, it may be more preferred that the article has a slit opening which has a hexagonal shape. An example of such a hexagonal shaped slit opening 30 is shown in Figure 2. The slit opening 30 contains a rectangular portion 90 and two triangular portions 91 at each side thereof. The length of the slit opening 30 is then measured from the joining point of the edges of the opening 30, in the top of the triangular 91 (i.e. the length of the longest dimension/ longitudinal axis of the hexagonal slit opening 30) and has the preferred values are as specified herein. The width of the hexagonal slit opening 30 is then the width of the transverse axis of this slit 30, orthogonal to the longitudinal axis of the slit opening 30.
The topsheet 24 comprises folds which may unfold when a low force, such as less than 1N is applied to the geometrical center point of the topsheet 24, typically by applying 1 N force on the middle point of an elasticated edge 32. The topsheet 24 can thus be extended in use. This ensures that when the backsheet 26 and core 28 become heavier due to the received bodily fluids, and start sagging downwards, the topsheet 24 can merely extend and remain in position, in close proximity to the wearer's skin.

Also, the limited attachment or no attachment of the topsheet 24 to the core 28 ensures that, when the diaper 20 receives bodily extudates and the core 28 and backsheet 26 are pulled downwards, due to the weight of the exudates received by the diaper 20, the topsheet 24 and the slit opening 30 do not move automatically with the core, but remain against the skin of the wearer, or in very close proximity to the wearer.
The diaper 20 also has leg cuffs 80 on both longitudinal edges of the diaper 20, typically attached to the backsheet 26. Preferred is that the longitudinal edge of a leg cuff 80, the longitudinal edge of the topsheet 24 and the longitudinal edge of the backsheet 26 are attached together in the form of a thin, longitudinal attachment edge.

### Test methods

### Force profile measurements

The topsheet/ cuff or article herein has a specific first load force (optionally a specific first unload force and second load force) and second unload forces at certain % elongation state or strain, as set out herein, and this is measured as follows. (It should be understood that 400% elongation sate or strain does not mean that the article is stretched to 400% of its original length, but that this represents an additional 400% elongation / strain of the grip to grip distance stretch, as set out below.)

If the elastic forces of the elasticated topsheet or cuff are to be measured, the topsheet/ cuff is removed from the rest of the article (such that the elastic forces are not impacted). This is then conditioned in relaxed state at 23°C and the 50% humidity for 16 hours. If the article has been packed or stored folded double or triple, the elasticated topsheet or cuffs should be unfolded 15 minutes prior to testing.
If the elastic forces of the article are to be measured, the article as a whole is conditioned in relaxed state at 23°C and the 50% humidity for 24 hours. If the article has been stored or packed folded double or triple, it should be unfolded 15 minutes prior to testing.

The following procedure is the same for the topsheet, cuff or article.
The article's (or topsheet's, or cuffs) transverse edges are each completely stretched in transverse direction to the full width, and then placed between one of a pair of opposing holders (see below), that are then placed between two grips in a vertical tensile tester Zwick type BX1120.25-013, as available from Zwick (Ulm, Germany). The holders have a length (corresponding with the width direction of the article, cuff or topsheet) of the maximum stretched width of the articles transverse edges, and a width (corresponding with the length direction of the article) of 50 mm. The sample holders are of Plexiglas of 1 mm thick, covered with Velcro hook material (as mentioned above).
The grips that are placed over the sample holders are 30 mm wide (in the length direction of the article) and 60 mm long (in the transverse, width direction of the article).

The load cell of the testing equipment is chosen such that the force results for the samples tested will be between 10% and 90% of the capacity of the load cell or load range used, i.e. for the diapers herein 50N.

The article, topsheet or cuff is in this test stretched to a maximum of 90% of total fully stretched length (this is at the 400% elongation or strain state) and this determines the grip to grip length at 0% strain/ elongation state. For example, for a diaper herein with a fully stretched article length of 450 mm, the grip to grip distance is chosen to be 61 mm, so that at 400% strain the article is stretched to a length of 405 mm (i.e. 90% of the maximum length of 450 mm), i.e. 305 mm (4 x 61 mm plus original 61mm) plus 2 x the grip width of 50 mm.

The measurement is done in a controlled environment, whereby the temperature is kept constant at 23°C and the humidity on 50%.

The test is started and the two cycle hysteresis is measured, using a 508 mm/min grip (crosshead) speed, by elongating the article, cuff or topsheet up to the 400% strain/ elongation state of the grip distance (e.g. 4 x 61 mm + original 61 mm) and relaxing it then immediately to the original position (e.g. 61 mm grip to grip distance), and reiterating this once more, for the second cycle.

The forces applied to the article at the various elongation points, e.g. 300% and 400% first load force, 150%, 200%, 300% first unload force, 100%, 150%, 200% 300% second unload force, are measured during the cycles ( i.e. first load, first unload, second load and second unload parts of the two cycles).
These values thus obtained are reported and referred to herein.

### Method to stretch the elastic region, elastic laminate portion herein to an elongation of ε= 0.5;

### Method to determine its fully stretched length Lₛ and εₘₐₓ

The elastic region or elastic laminate portion may be straight, curved, or it may comprise several straight parts that are joined under one or more angles with one another, as can been seen in Figures 1 and 2, or it may have a combination of such configurations. This is herein referred to, respectively, as "straight", "curved" or "angled" elastic laminate portion, respectively, or for example, "curved and angled" elastic laminate portion etc.
In each case below, a sample (e.g. cuff or preferably an elastic laminate portion thereof, if this can be isolated as such) is obtained from an absorbent article that has been conditioned for 24 hours at 50% humidity and 23°C.

### 1) When the elastic region/ elastic laminate portion is straight:

The elastic laminate portion or cuff as a whole is obtained and put on a flat surface as described above to measure the contracted length of the elastic laminate portion L_{c}.

The elastic laminate portion is subsequently elongated to 1.5 L_{c} (equals ε =0.5) or to its fully stretched length Lₛ, to determine εₘₐₓ as follows.

The sample (the cuff with the elastic laminate portion or the elastic laminate portion thereof) is left for 24 hours at 25°C and 50% humidity, prior to the elongation/ stretching step below, which is subsequently performed under the same conditions.

Measurement of lengths of the samples can be done with a micrometer screw.

The sample to be tested is placed length-wise (in the direction of stretch) between two tweezers or, if the width of the sample is more than 1 cm, between two clamps of a width of 1 cm, one on each end, such that contact area of the tweezers/ clamp and the sample is at the most 1 mm for clamps and 0.5mm for tweezers in the direction of stretch (length). The exact distance between the start of one clamp or tweezers to the beginning of the other clamp or tweezers is measured. This is the contracted length of the sample, e.g. of the laminate portion.
For straight samples, the clamps or tweezers are moved in the y-direction of the length of the straight samples, such that the length direction is the direction of the elongation force.

The sample may thus be stretched to its maximum elongation (e.g. when the cuff/ topsheet reaches its maximum length) and the length of the sample and the distance between the clamps is measured, and the elongation εₘₐₓ is calculated.

Alternatively, the sample is stretched to ε = 0.5, in order to submit the thus stretched sample to the Primos method set out below.

### 2) When the elastic region/ elastic laminate portion is "angled":

The elastic laminate portion is divided by marking with a fine marker pen into straight parts (i.e. between the angles), for example in 3 straight parts. The sample is prepared and conditioned as described above.
Subsequently, each straight part is elongated separately by the method set out above for straight elastic laminate portions to either ε = 0.5 or εₘₐₓ, e.g. when the sample comprises two angles and 3 straight parts, 3 force lines are determined and the sample is stretched 3 steps.

### 3) When the elastic region/ elastic laminate portion is curved:

The curved elastic laminate portion is divided with a fine marker pen into sections of 2 cm absolute length and possibly one remaining section of a smaller length. The sample is prepared and conditioned as described above.

Prior to elongation, the force line of each section of 2 cm (or one section of less than 2 cm) is determined as follows. Each section has two transverse edge lines that are 2 cm apart, and each transverse edge line has a centre point. A line can be drawn through said two points of said two transverse edge lines. This will be the "y-direction line" or force line along which the force will be applied to elongate said section. This will be done for each section.

Subsequently, each section is elongated separately by the manner set out above for straight elastic laminate portions, but by separately elongating each section along its own force line, to either ε = 0.5 or its maximum elongation εₘₐₓ.
After stretching all sections, a fully stretched absolute length can be measured for each section and for the elastic laminate portion, Lₛ and εₘₐₓ can be calculated.

### 4) Mixed elastic regions/ elastic laminate portions

If the elastic laminate portion comprises a combination of curved, angled and/ or straight parts, then a combination of the above methods is applied accordingly.

### Primos method:

### Determination of the wrinkle heights and density, averages and deviations thereof

The following described the method to determine the wrinkle height and winkle density of the laminate portion of the cuff/ topsheet
Each sample with the elongation of 0.5 as defined and obtained by the method described herein, is examined by use of PRIMOS equipment and its data acquisition software, following the manufacture's instructions manual, using a 13x18mm lens.
If the elastic laminate portion has an average width of more than 3 mm, then the measurement above is only done on the inner 70% of the width of the laminate portion, along its length.

The PRIMOS equipment will provide graphs per measured section of the sample , as shown in Figure 4, and it provides exact values per wrinkle, e.g. height, width, and it allows to calculate the average of wrinkles per cm, wrinkle height, deviations etc.

### Caliper measurement

The caliper and average caliper of an elastic laminate portion or regions that has an elongation of ε= 0.5, as described herein, can be obtained by use of a micrometer, such as the Frank 16303, obtainable from Twing Albert-Frank GmbH. The test is done at 23 °C, 50% humidity. The sample should be already conditioned to this humidity and temperature as set out above, since it has been conditioned for 24 hours under these conditioned, prior to stretching it to the required elongation of 0.5, needed to do this caliper test. The equipment is calibrated prior to testing. The lowering speed of the pressure foot is set to be 3 mm/ sec and the dwelling time 2-5 sec.

The size (surface area) of the anvil is chosen depending on the size of the elastic laminate potion and subsequently the weight on the pressure foot is chosen such that the required pressure of 0.33psi is obtained.

For example, an anvil with a 40 mm diameter is used and a total weight of 295 grams (80 grams of the pressure foot plus an additional 115 grams) is applied to measure preferred elastic laminate portion(s) herein.

To obtain the average caliper of the elastic laminate portion, the test is repeated on several portions of the elastic laminate portion, such that the areas pressed by the anvil per measurement do not overlap. Subsequently, an average can be calculated. Also the deviation in the caliper can be calculated.

### Handle-o-meter bending rigidity test

This method serves to determine the bending rigidity (and thereby softness) of a nonwoven layer or nonwoven sheet used for the cuff or topsheet herein, as described herein, and reflects the flexibility and surface friction of the material. In this test, a nonwoven is deformed through a slot by use of a plunger, and the required force is measured. This method is based on the INDA Standard test IST 90.3-92

A sample material of the nonwoven sheet or nonwoven layer of 1 inch long and 1 inch wide (25mm x 25mm) is cut and conditioned at 65% humidity and 21°C as set out in the INDA test. The sample is free form elastic material or edges attached to other materials. In one embodiment, the average fiber direction of the nonwoven web or layer in contact with the skin in use can be determined and this would be the Y direction (e.g. in use typically corresponding MD dimension of the absorbent article).
A handle-o-meter, available from Twingh- Albert Instruments Co., Philadelphia, USA, is calibrated as set out its user instructions.
The slot width is 6.35mm.
The sample is placed under the plunger and on the slot with the surface that in use contacts or faces the skin up wards facing up. A first dimension is perpendicular to the slot and this is the direction tested, for which the bending rigidity is reported herein. In one embodiment, this is the average fiber direction of the skin-facing surface, e.g. the spunbond layer. The sample is centered over the slot and the test is run and the force is measured. This value is *multiplied by 4* (e.g. normalised to a 4 inch x 4 inch sample) and reported in grams herein as the bending rigidity.

### Hydrostatic head (hydrohead)

The hydrostatic head (also referred to as hydrohead) as used herein is measured with a low surface tension liquid, i.e. a 49 mN/m liquid (solution).
This liquid is prepared as set out below.
This test is performed as set out in co-pending application W02005/112854A, conform the Inda/ Edana test WSP 80.6 (05). However, the water pressure (from below) is increased with a rate is 60 mbar/min.

A sample of 5 cm² is taken from the nonwoven sheet or cuff or topsheet herein. The sample should be free from elastic material or edges that are connected to other materials.
The test head used has a 2.5 cm diameter; the protective sleeve used has a 2.2 cm diameter.
The test is performed on this sample and the Hydrostatic head value is obtained, and referred to herein.

### 49 mN/m (dynes/ cm) liquid preparation:

A 10 litre canister with tap is cleaned thoroughly 3 times with 2 litres polyethylene and then 3 times with 2 litres distilled/deionized water.
Then, it is filled with 10 litres distilled/deionized water and stirred with a clean stirring bar for 2 h, after which the water is released via the tap.
A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.
The stirring bar is removed from the glass with a magnetic stick (without touching the solution) and then the Na cholate solution is poured into the 10 litres canister and more distilled/deionized water is added such that the concentration of the final solution is 3 g/l. This is further stirred with a stirring bar for 2 hours and then used.
This preparation of the solution and use thereof is at the temperature stated for the test for which it is used, or if no temperature is stated, it is kept at 20°C.

The surface tension of the solution is measured and this should be 49 mN/m (+/- 2). (The surface tension may be determined by method: ASTM D1331-56 ("Standard test method for surface and interfacial tension of solution of surface active agents") using a Kruss K12 tensiometer.)

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article having a backsheet, an absorbent core and an elasticated topsheet, comprising elasticated regions or an elasticated anal and/ or genital cuff, comprising elasticated regions, said topsheet or cuff defining at least one opening to receive fecal material, said article, cuff, topsheet and opening having a longitudinal direction, length and longitudinal axis (Y), and a transverse direction, width and transverse axis (X),
whereby in 100% stretched state said article has a length (in longitudinal direction) of 47 cm or less, and whereby said elasticated cuff or topsheet or said article has a second unload force at 150% elongation state of at least 0.10N and a second unload force at 200% elongation state of at least 0.20N, said second unload force at 200% elongation being less than 0.9N.

2. An absorbent article as in claim 1 whereby said absorbent article has a second unload force at 150% elongation state of at least 0.15N and a second unload force at 200% elongation state of from 0.30N to less than 0.8N.

3. An absorbent article as in claim 1 or 2 whereby said cuff has a first load force at 300% elongation state of less than 3.0N, preferably less than 2.8 N or less than 2.7N.

4. An absorbents article as in any preceding claim whereby said length of the article is 46 cm or less, preferably 37 to 45 cm.

5. An absorbent article as in any preceding claim, whereby said absorbent core a has a width, measured along the centre transverse line or x-axis of the article, of from 7 to 10 cm, preferably 7.5 to 9.5 cm.

6. An absorbent article as in any preceding claim, whereby said cuff or topsheet comprises a barrier nonwoven sheet, having at least two nonwoven layers that are not fully laminated and that comprie each at least two nonwoven webs that are laminated.

7. An absorbent article as in any preceding claim, whereby said cuff or topsheet comprises a nonwoven sheet having a bending rigidity of less than 25 grams.

8. An absorbent article as in any preceding claim, whereby said cuff or topsheet has a longitudinally extending opening with substantially parallel longitudinal side edges and whereby said cuff or topsheet comprises at least two elastic regions each formed by a longitudinally extending elastic bands or strands, said at least two elastic bands or strands being partially parallel to one another, at least one being positioned along at least part of a first longitudinal edge of the opening and at least one being positioned along at least part of the other longitudinal edge of the opening.

9. An absorbent article as in claim 8, whereby said elastic bands or strands extend beyond the opening's transverse edges to or towards the transverse edge of the article, whereby two opposing elastic bands or strands diverge from one another in each area beyond the transverse edge of the opening.

10. An absorbent article as in any preceding claim, whereby the transverse edge of the absorbent article is free of any elastic material that has transverse stretch.

11. An absorbent article as in any preceding claim, comprising at least a pair of opposing elasticated regions, with each a middle portion and each a front and back end portion, said middle portions being substantially parallel to one another, whist the front and back end portions (in relaxed state) bend away from one another (in the plane of the topsheet/ cuff), whereby the distance between the opposing front end portions of the elastic regions is larger that the distance between the opposing middle portions and whereby the distance between the opposing back end portions of the elastic regions is larger that the distance between the middle portions of the elastic regions.

12. An absorbent article as in claim 11, whereby said two front end portions and the two back end portions of the elasticated regions each have an angle with the longitudinal axis of the opening of from 15° and 35°, preferably from 20° to 30°, or preferably from 24° and 28°.

13. An absorbent article as in any preceding claim, comprising a sub-layer for feces storage or immobilisation between said cuff or topsheet and the absorbent core.

14. An article as in any preceding claim, which is baby diaper suitable for babies of 2 to 9 kg.
